# EUROPEAN PATENT SPECIFICATION

(11) **EP 2 510 924 B1**
(45) Date of publication and mention of the grant of the patent: **08.10.2014**
(21) Application number: 09852087.7
(22) Date of filing: 08.12.2009
(51) Int. Cl.: A61K 9/48, A61K 9/14, A61K 9/16, A61K 9/20, A61K 31/7032, A61P 35/00

(54) **SOLID DISPERSIONS CONTAINING 20-O-ß-D-GLUCOPYRANOSYL-20(S)-PROTOPANAXADIOL**
FESTE DISPERSIONEN MIT 20-O-ß-D-GLUCOPYRANOSYL-20(S)-PROTOPANAXADIOL
DISPERSIONS SOLIDES CONTENANT DU 20-O-ß-D-GLUCOPYRANOSYL-20(S)-PROTOPANAXADIOL

(43) Date of publication of application: 17.10.2012
(73) Proprietor: IL Hwa Co., Ltd, Guri-si, Gyeonggi-do 471-816 (KR)
(72) Inventor: LEE, Sung Kyun, Seoul 150-933 (KR); PARK, Yong-Duck, Seoul 135-785 (KR); JANG, Jae Won, Seoul 132-792 (KR); HUR, Bong Hang, Suwon-si Gyeonggi-do 442-882 (KR); CHI, Sang Cheol, Suwon-si Gyeonggi-do 443-737 (KR)
(74) Representative: Von Kreisler Selting Werner - Partnerschaft von Patentanwälten und Rechtsanwälten mbB
(86) International application number: PCT/KR2009/007305
(87) International publication number: WO 2011/071194

(56) References cited:
- KR-B1- 0 173 349
- KR-B1- 100 730 719
- KR-B1- 100 767 349
- DATABASE WPI Week 200481 Thomson Scientific, London, GB; AN 2004-814632 XP002711980, & CN 1 526 404 A (SHANDONG LUYE NATURAL MEDICINE RES DEV C) 8 September 2004 (2004-09-08)
- DATABASE WPI Week 200737 Thomson Scientific, London, GB; AN 2007-391216 XP002711981, & CN 1 879 647 A (HANGZHOU INNOVATION CHINESE MEDICINE STA) 20 December 2006 (2006-12-20)
- LEE, P.S. ET AL.: 'Physicochemical characteristics and bioavailability of a novel intestinal metabolite of ginseng saponin (IH901) complexed with beta-cyododextrin' INTERNATIONAL JOURNAL OF PHARMACEUTICS vol. 316, 2006, pages 29 - 36, XP027972408

## Description

### Technical Field

The present invention relates to a solid dispersion containing 20-O-β-D-glucopyranosyl-20(S)-protopanaxadiol.

### Background Art

20-O-β -D-glucopyranosyl-20(S)-protopanaxadiol is a metabolite of ginsenoside created by intestinal microbes, and is a material having great anticancer effect.

Currently, most anticancer drugs used in cancer treatment are chemotherapeutic agents, which intervene in various metabolic pathways and directly act on DNA to inhibit the replication, transcription and translation of DNA, to prevent the synthesis of nucleic acid precursors and to control cell division, thus exhibiting anticancer activity, that is, cell toxicity to cancer cells. However, these chemotherapeutic agents have toxicity to normal cells as well as cancer cell. Therefore, these chemotherapeutic agents produce harmful side effects on undesired cells, such as normal cells. Although these chemotherapeutic agents have stronger toxicity to rapidly proliferating cancer cells than they have toxicity to normal cells, they may cause unfavorable side effects on the human body because new cells actively grow in the human body. Since bone marrow, hair follicles, endothelial cells of the stomach and intestines, and the like, in which cell proliferation actively occurs, are greatly influenced by these chemotherapeutic agents, these chemotherapeutic agents cause harmful side effects on the immune-related cells produced from the bone marrow of patients undergoing drug therapy. That is, side effects, such as bacterial infection, natural bleeding, hair loss, nausea, vomiting and the like, are caused by the reduction of immune-related leukocytes, the reduction of blood coagulation-related erythrocytes, leukocytes and thrombocytes, and the like.

In order to solve the side effects of chemotherapeutic agents, various kinds of anticancer drugs have been developed. Among these anticancer drugs, natural substance-derived anticancer drugs are being developed the most actively.

20-O-β-D-glucopyranosyl-20(S)-protopanaxadiol, which is a metabolite of ginsenoside, is one of such natural substance-derived anticancer drugs, and is known to have little side effects and have a drug efficacy equal to or higher than those of conventional anticancer drugs. Korean Patent registration Nos. 0164266 and 0178863 (Patentee: Ilhwa Co., Ltd.) disclose the anticancer use of 20-O-β-D-glucopyranosyl-20(S)-protopanaxadiol and a method of preparing 20-O-β-D-glucopyranosyl-20(S)-protopanaxadiol. Further, Korean Patent registration No. 10-0412218 (Patentee: Ilhwa Co., Ltd.) discloses that 20-O-β-D-glucopyranosyl-20(S)-protopanaxadiol is briefly designated as "IH-901".

Ginsenoside, which is an important active material in ginseng, serves to increase cholesterol metabolism, accelerate the synthesis of serum protein, improve immunity and activate anti-inflammation. Ginsenoside (Rb1, Rb2, Rc or the like) is converted into 20-O-β-D-glucopyranosyl-20(S)-protopanaxadiol (hereinafter, referred to as "IH-901") by microbes in intestines of the human body. The IH-901 functions to prevent cancer cells from intruding into the human body and to inhibit cancer cells from being transferred by controlling the formation of tumors and anomalies in the chromosomes.

However, at the time of the oral administration of IH-901, the bioavailability of IH-901 is very low (3.5%) [refer to physicochemical characteristics and bioavailability of a novel intestinal metabolite of ginseng saponin (IH901) complexed with β-cyclodextrin, International Journal of Pharmaceutics 2006, 316, 29-36], and thus it cannot be expected that anticancer action is sufficiently exhibited after its oral administration. The reason for this is that the dissolution rate of this drug is low after it has been orally administered because this drug is poorly-soluble to water. In order to solve such a problem, there has been research into improving the solubility of this drug in water and the elution rate thereof.

The paper "Solubilization of IH-901, a Novel Intestinal Metabolite of Ginseng Saponin, in Aqueous Solution", (Journal of Pharmaceutical Investigation, 2004, 34, 385-391) reported that when a cosolvent (ethanol, propylene glycol, polyethylene glycol 300, polyethylene glycol 400 or glycerin), which is a typical solubilizer, a surfactant (polysorbate 80, polyoxy 35 castor oil, polyoxyl 40 hydrogenated caster oil, poloxamer 188 or poloxamer 407), or an inclusion compound forming agent (hydroxypropyl-β-cyclodextrin) was used in order to improve the solubility of poorly-soluble IH-901 in water, the solubility of IH-901 in an aqueous solution including 40% of an cosolvent and 10% of a surfactant or an inclusion compound forming agent (hydroxypropyl-β-cyclodextrin) was 10 mg/ml or less. The result may not be sufficient solubility necessary for oral formulation.

Further, in the dissolution test of the inclusion compound of IH-901, prepared using β-cyclodextrin as an inclusion compound forming agent, using water as an dissolution medium, it was ascertained that the dissolution rate of IH-901 was about 10% after 6 hours, and thus IH-901 was not sufficiently solubilized, and that, when this inclusion compound was orally administered, the bioavailabilty thereof was measured, and, as a result, the bioavailability thereof was increased about two times of that of IH-901 powder, but the bioavailability thereof was 6.6%, which was also low [refer to "physicochemical characteristics and bioavailability of a novel intestinal metabolite of ginseng saponin (IH901) complexed with β-cyclodextrin", International Journal of Pharmaceutics 2006, 316, 29-36].

Therefore, in order to obtain sufficient drug efficacy at the time of oral administration of IH-901, it is required to develop technologies for increasing the dissolution rate of this drug.

### Disclosure

### Technical Problem

Accordingly, the present invention has been made to solve the above-mentioned problems, and an object of the present invention is to provide a solid dispersion having a high 20-O-β-D-glucopyranosyl-20(S)-protopanaxadiol dissolution rate.

### Technical Solution

The present invention provides a solid dispersion, including: 20-O-β-D-glucopyranosyl-20(S)-protopanaxadiol (hereinafter, referred to as "IH-901") which is a pharmacologically active ingredient; and a saturated polyglycolized glyceride which is a lipid matrix.

The saturated polyglycolized glycerides are mixtures of mono-, di- and tri-glycerides and polyethylene glycol mono-and di-esters obtained either by partial alcoholysis of hydrogenated vegetable oils using polyethylene glycol of relative molecular weight ranging from 200-2000, or by esterification of saturated fatty acids using polyethylene glycol of relative molecular weight ranging from 200-2000 and glycerol(cited in French Pharmacopeia 10th Edition, and recited in WO 95/07696). Gelucire (commercially available from Gattefosse s.a., Saint Priest, France) is sold in the market as a typical good of the saturated polyglycolized glyceride. Examples of Gelucire include Gelucire 35/10, Gelucire 44/14, Gelucire 46/07, Gelucire 50/13, Gelucire 53/10 and the like, each of which is characterized by melting point and HLB (hydrophilic-lipophilic balance). Each Gelucire is designated by two numbers separated by a slash, the first number(two-digit number) indicating its melting point and the second, the HLB (hydrophilic-lipophilic balance). For instance, in Gelucire 44/14, the melting point thereof is 44°C, and the HLB (hydrophilic-lipophilic balance) thereof is 14.

Drop point, hydroxyl value, saponification value and fatty acid composition of each Gelucire are as follows.

### Gelucire 35/10

drop point: 29 - 34°C (preferably, 31.2°C)
hydroxyl value: 70 - 90mg KOH/g (preferably, 74mg KOH/g)
saponification value: 120 ~ 134mg KOH/g (preferably, 134mg KOH/g)
fatty acid composition:
caprylic acid (C8): 1 ~ 7% (preferably, 2.1%)
capric acid (C10): 1 ~ 7% (preferably, 2.2%)
lauric acid (C12): 31 ~ 41% (preferably, 35.4%)
myristic acid (C14): 7 ~ 17% (preferably, 12.9%)
palmitic acid (C16): 12 ~ 22% (preferably, 20.7%)
stearic acid (C18): 23 ~ 33% (preferably, 26.2%)

### Gelucire 44/14

drop point: 42.5 ~ 47.5°C
hydroxyl value: 30 ~ 50mg KOH/g
saponification value: 76 ~ 90mg KOH/g
fatty acid composition:
caprylic acid (C8): 4 ~ 10%
capric acid (C10): 3 ~ 9%
lauric acid (C12): 40 ~ 50%
myristic acid (C14): 14 ~ 24%
palmitic acid (C16): 4 ~ 14%
stearic acid (C18): 5 ~ 15%

### Gelucire 46/07

drop point: 47 ~ 52°C (preferably, 49.3°C)
hydroxyl value: 65 ~ 85mg KOH/g (preferably, 74mg KOH/g)
saponification value: 126 ~ 140mg KOH/g (preferably, 139mg KOH/g)
fatty acid composition:
caprylic acid (C8): < 3% (preferably, <0.1%)
capric acid (C10): < 3% (preferably, <0.1%)
lauric acid (C12): < 5% (preferably, 0.9%)
myristic acid (C14): < 5% (preferably, 1.4%)
palmitic acid (C16): 40 - 50% (preferably, 44%)
stearic acid (C18): 48 - 58% (preferably, 52.8%)

### Gelucire 50/13

drop point: 46 - 51°C (preferably, 48.7°C)
hydroxyl value: 36 - 56mg KOH/g (preferably, 52mg KOH/g)
saponification value: 67 - 81mg KOH/g (preferably, 74mg KOH/g)
fatty acid composition:
caprylic acid (C8): < 3% (preferably, 0.2%)
capric acid (C10): < 3% (preferably, 0.2%)
lauric acid (C12): < 5% (preferably, 2.2%)
myristic acid (C14): < 5% (preferably, 1.8%)
palmitic acid (C16): 40 - 50% (preferably, 42.5%)
stearic acid (C18): 48 - 58% (preferably, 52.6%)

### Gelucire 53/10

drop point: 49 - 54°C (preferably, 52.5°C)
hydroxyl value: 25 - 45mg KOH/g (preferably, 35mg KOH/g)
saponification value: 98 - 112mg KOH/g (preferably, 104mg KOH/g)
fatty acid composition:
caprylic acid (C8): < 3% (preferably, < 0.1%)
capric acid (C10): < 3% (preferably, < 0.1%)
lauric acid (C12): < 5% (preferably, 0.4%)
myristic acid (C14): < 5% (preferably, 1.0%)
palmitic acid (C16): 40 - 50% (preferably, 43%)
stearic acid (C18): 48 - 58% (preferably, 54.2%)

The solid dispersion of the present invention is obtained in the form of IH-901 being dispersed in saturated polyglycolized glyceride which is a lipid matrix.

The solid dispersion may include the saturated polyglycolized glyceride in an amount of 200 - 5000 parts by weight based on 100 parts by weight of the IH-901. When the amount of the saturated polyglycolized glyceride is less than 200 parts by weight, the IH-901 cannot be sufficiently solubilized. Further, when the amount thereof is more than 5000 parts by weight, it is difficult to formulate the solid dispersion due to the excessive use thereof.

In the solid dispersion of the present invention, a preferable example of the saturated polyglycolized glyceride is Gelucire 44/14. This excipient is called "lauroyl polyoxyl-32 glyceride", which is compendial product of the United states Phamacopoeia/National Formulary(USP/NF).

The solid dispersion may be prepared by dispersing IH-901 in the saturated polyglycolized glyceride using a solvent method, a melting method, a mixing method or the like, which is generally used to prepare solid dispersions.

Moreover, the solid dispersion may be formed into a capsule formulation by directly encapsulating the solid dispersion or by adding a pharmaceutically acceptable excipient to the solid dispersion and then encapsulating the mixture, and may be formed into a tablet formulation by mixing a pharmaceutically acceptable excipient with the solid dispersion and then tableting the mixture to give a tablet. Examples of pharmaceutically acceptable excipient may include: diluents, such as starch, lactose, microcrystalline cellulose, calcium hydrogen phosphate and the like; and/or talc, silicon dioxide, stearic acid, and metal salts thereof; and/or lubricants, such as magnesium aluminosilicate and the like.

The solid dispersion may be formed into a capsule formulation or a tablet formulation. The solid dispersion may be formed into a capsule formulation by filling only the solid dispersion in a soft capsule or a hard capsule or filling both the solid dispersion and the excipient in a hard capsule or a soft capsule, and may be formed into a tablet formulation by tableting the solid dispersion together with the excipient.

Specifically, the solid dispersion may be formed into a capsule formulation by directly filling the solid dispersion in a soft capsule or a hard capsule using a general capsule filling machine or a liquid filling machine or by adding an excipient to the solid dispersion and then filling the mixture in a soft capsule or a hard capsule using a general capsule filling machine or a liquid filling machine. Further, the solid dispersion may be formed into a tablet formulation by adding an excipient, such as a diluent or a lubricant, to the solid dispersion and then pulverizing the mixture to form a powder and then tableting the powder, or may be formed into a capsule formulation by filling the powder in a hard capsule or a soft capsule.

Therefore, the present invention provides the solid dispersion wherein formulation of the solid dispersion is a capsule formulation.

Further, the present invention provides a pharmaceutical formulation, including: the solid dispersion of the present invention; and a pharmaceutically acceptable excipient. For example, the pharmaceutical formulation may be a capsule formulation or a tablet formulation.

### Advantageous Effects

The solid dispersion of the present invention has an effects of increasing the dissolution rate of IH-901.

### Description of Drawings

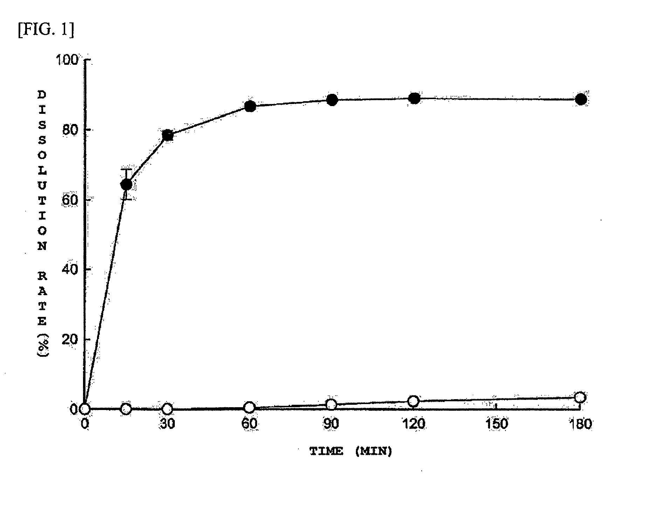

FIG. 1 is a graph showing the result of dissolution test of the solid dispersion prepared in Example 2.

### Mode for Invention

Hereinafter, the present invention will be described in more detail with reference to the following Examples. These Examples are set forth to illustrate the present invention, and the scope of the present invention is not limited thereto.

### <Example 1> Preparation of a solid dispersion of IH-901 dispersed in saturated polyglycolized glyceride using a solvent method

IH-901: 100 parts by weight
Gelucire 44/14: 200 parts by weight

100 g of IH-901 (manufactured by Ilhwa Co., Ltd., Gurisi, Korea) and 200 g of Gelucire 44/14 (manufactured by Gattefosse Corp., France) were completely dissolved in 800 mL of a mixed solvent of dichloromethane: ethanol (60: 40) to form a solution. This solution is heated to 60°C to volatilize the mixed solvent, thereby preparing a solid dispersion.

### <Example 2> Preparation of a solid dispersion of IH-901 dispersed in saturated polyglycolized glyceride using a melting method

IH-901: 100 parts by weight
Gelucire 44/14: 500 parts by weight

500 g of Gelucire 44/14 was heated to 60°C to completely melt the Gelucire 44/14, and then 100 g of IH-901 was added to the molten Gelucire 44/14 and then heated to 150°C to completely melt the mixture of Gelucire 44/14 and IH-901. Thereafter, the molten mixture was cooled to room temperature, thereby preparing a solid dispersion.

### <Example 3> Preparation of a solid dispersion of IH-901 dispersed in saturated polyglycolized glyceride using a melting method

IH-901: 100 parts by weight
Gelucire 44/14: 1000 parts by weight

1000 g of Gelucire 44/14 was heated to 60°C to completely melt the Gelucire 44/14, and then 100 g of IH-901 was added to the molten Gelucire 44/14 and then heated to 130°C to completely melt the mixture of Gelucire 44/14 and IH-901. Thereafter, the molten mixture was cooled to room temperature, thereby preparing a solid dispersion.

### <Example 4> Preparation of a solid dispersion of IH-901 dispersed in saturated polyglycolized glyceride using a melting method

IH-901: 100 parts by weight
Gelucire 44/14: 2000 parts by weight

200 g of Gelucire 44/14 was heated to 60°C to completely melt the Gelucire 44/14, and then 10 g of IH-901 was added to the molten Gelucire 44/14 and then heated to 130°C to completely melt the mixture of Gelucire 44/14 and IH-901. Thereafter, the molten mixture was cooled to room temperature, thereby preparing a solid dispersion.

### <Example 5> Preparation of a solid dispersion of IH-901 dispersed in saturated polyglycolized glyceride using a melting method

IH-901: 100 parts by weight
Gelucire 44/14: 5000 parts by weight

500 g of Gelucire 44/14 was heated to 60°C to completely melt the Gelucire 44/14, and then 10 g of IH-901 was added to the molten Gelucire 44/14 and then heated to 130°C to completely melt the mixture of Gelucire 44/14 and IH-901. Thereafter, the molten mixture was cooled to room temperature, thereby preparing a solid dispersion.

### <Example 6> Preparation of a solid dispersion of IH-901 dispersed in saturated polyglycolized glyceride using a melting method

IH-901: 100 parts by weight
Gelucire 44/14: 500 parts by weight
Magnesium aluminosilicate: 300 parts by weight

500 g of Gelucire 44/14 (manufactured by Gattefosse Corp., France) was heated to 60°C to completely melt the Gelucire 44/14, and then 100 g of IH-901 was added to the molten Gelucire 44/14 and then heated to 150°C to completely melt the mixture of Gelucire 44/14 and IH-901. Thereafter, the molten mixture was cooled to room temperature, and then 300g of magnesium aluminosilicate (Neusilin, manufactured by Fuji Chemicals Industry Co., Ltd.) was added thereto and then cooled to room temperature, thereby preparing a solid dispersion having excellent fluidity.

### <Example 7> Preparation of a solid dispersion of IH-901 dispersed in saturated polyglycolized glyceride using a melting method

IH-901: 100 parts by weight
Gelucire 50/13: 2000 parts by weight

200 g of Gelucire 50/13 (manufactured by Gattefosse Corp., France) was heated to 65°C to completely melt the Gelucire 50/13, and then 10 g of IH-901 was added to the molten Gelucire 50/13 and then heated to 130°C to completely melt the mixture of Gelucire 50/13 and IH-901. Thereafter, the molten mixture was cooled to room temperature, thereby preparing a solid dispersion.

### <Example 8> Preparation of a solid dispersion of IH-901 dispersed in saturated polyglycolized glyceride using a melting method

IH-901: 100 parts by weight
Gelucire 50/13: 5000 parts by weight

500 g of Gelucire 50/13 (manufactured by Gattefosse Corp., France) was heated to 65°C to completely melt the Gelucire 50/13, and then 10 g of IH-901 was added to the molten Gelucire 50/13 and then heated to 130°C to completely melt the mixture of Gelucire 50/13 and IH-901. Thereafter, the molten mixture was cooled to room temperature, thereby preparing a solid dispersion.

### <Example 9> Manufacture of a capsule formulation

The solid dispersion prepared in Example 2 was filled in a hard capsule III at a rate of 150 mg per capsule using a liquid filling machine (FS03-SN008, manufactured by Nosakatech Corp., Japan) to manufacture a capsule formulation.

### <Example 10> Manufacture of a capsule formulation

The solid dispersion prepared in Example 4 was filled in a hard capsule I at a rate of 420 mg per capsule using a liquid filling machine to manufacture a capsule formulation.

### <Example 11> Manufacture of a capsule formulation

The solid dispersion prepared in Example 6 was filled in a hard capsule I at a rate of 420 mg per capsule using a manual capsule filling machine (manufactured by Kumsung Machinery Co., Ltd., Korea) to manufacture a capsule formulation.

### <Example 12> Manufacture of a tablet formulation Solid dispersion prepared in Example 6: 180 mg

Anhydrous lactose: 176 mg
Magnesium stearate: 4 mg

180 mg of the solid dispersion prepared in Example 6 was mixed with 176 mg of anhydrous lactose for 20 minutes to form a first mixture, and then 4 mg of magnesium stearate was mixed with the first mixture for 5 minutes to form a second mixture, and then the second mixture was tableted at a rate of 360 mg per tablet using a tableting machine (Mini Press-II SF, manufactured by Rimek Co., Ltd., Indo) to manufacture a tablet formulation.

### <Test Example 1> dissolution test

The dissolution profile of a drug from the solid dispersion prepared in Example 2 was evaluated. IH-901, which had been used to prepare the solid dispersion of Example 21, was used as a control group. The dissolution test was carried out under the conditions that the second method (paddle method) of the dissolution test methods in the general test method of the United states Phamacopoeia was used, 900 mL of purified water was used as a test liquid, and the rotational speed of a paddle was 50 rpm. 25 mg of IH-901 (control group) or the solid dispersion prepared in Example 2 corresponding to 25 mg of IH-901 was dissolved in a dissolution liquid, left for 180 minutes and then filtered. Then, the content of IH-901 in the filtered solution was measured by high performance liquid chromatography. The high performance liquid chromatograph is commercially available from Hitachi Co., Ltd. The analysis of high performance liquid chromatography was conducted under the conditions of a C18 column (Luna, 5 um, 250 mm, Phenomenex Corp., U.S.A), mobile phase of acetonitrile: water (70: 30), a flow rate of 1 mL/min, a detection wavelength of 205 nm and an injection volume of 50 ul. Sample sizes were three respectively, and the dissolution test results thereof are shown in FIG. 1. In FIG. 1, the X-axis represents time passage, and the Y-axis represents dissolution rate (%). Further, in FIG. 1, the raw material is represented as ○, and the solid dispersion prepared in Example 2 is represented by ●.

As a result, a drug was scarcely dissolved from IH-901 (raw material), and the dissolution rate of the drug from IH-901 was about 3% even after 3 hours. However, the dissolution rate of a drug from the solid dispersion prepared in Example 2 was 65% after 15 minutes, and was about 90% after 3 hours.

Therefore, it can be seen that the solid dispersion of the present invention have effects of significant increase in the dissolution rate of IH-901.

### Industrial Applicability

The present invention provides a solid dispersion, including: 20-O-β -D-glucopyranosyl-20(S)-protopanaxadiol which is a pharmacologically active ingredient; and a saturated polyglycolized glyceride which is a lipid matrix. The solid dispersion has effects of increasing the dissolution rate of 20-O-β -D-glucopyranosyl-20(S)-protopanaxadiol. Therefore, the solid dispersion has industrial applicability.

## Claims

1. A solid dispersion, comprising:
20-O-β-D-glucopyranosyl-20(S)-protopanaxadiol which is a pharmacologically active ingredient; and
a saturated polyglycolized glyceride which is a lipid matrix.

2. The solid dispersion of claim 1, wherein the saturated polyglycolized glyceride is a mixture of mono-, di- and tri-glycerides and polyethylene glycol mono- and di-esters.

3. The solid dispersion of claim 1, wherein the solid dispersion comprises: 100 parts by weight of the 20-O-β-D-glucopyranosyl-20(S)-protopanaxadiol; and 200 - 5000 parts by weight of the saturated polyglycolized glyceride.

4. The solid dispersion of claim 1, wherein the saturated polyglycolized glyceride is lauroyl polyoxyl-32 glyceride.

5. The solid dispersion of claim 1, wherein formulation of the solid dispersion is a capsule formulation.

6. A pharmaceutical formulation, comprising: the solid dispersion of claim 1; and a pharmaceutically acceptable excipient.

7. The pharmaceutical formulation of claim 6, wherein the formulation is a capsule or tablet formulation.

8. The solid dispersion of claim 1, wherein the solid dispersion comprises: 100 parts by weight of the 20-O-β-D-glucopyranosyl-20(S)-protopanaxadiol; and 200 - 5000 parts by weight of the saturated polyglycolized glyceride; and
wherein the saturated polyglycolized glyceride is lauroyl polyoxyl-32 glyceride.

9. A pharmaceutical formulation, comprising: the solid dispersion of claim 3; and a pharmaceutically acceptable excipient.

## Patentansprüche

1. Feste Dispersion, umfassend:
20-O-β-D-Glucopyranosyl-20(S)-protopanaxadiol als pharmakologischen Wirkstoff; und
ein gesättigtes polyglycolisiertes Glycerid als Lipidmatrix.

2. Feste Dispersion gemäß Anspruch 1, wobei das gesättigte polyglycolisierte Glycerid ein Gemisch von Mono-, Di- und Triglyceriden sowie Polyethylenglycolmono- und -diestern ist.

3. Feste Dispersion gemäß Anspruch 1, wobei die feste Dispersion Folgendes umfasst: 100 Gewichtsteile des 20-O-β-D-Glucopyranosyl-20(S)-protopanaxadiols und 200 bis 5000 Gewichtsteile des gesättigten polyglycolisierten Glycerids.

4. Feste Dispersion gemäß Anspruch 1, wobei es sich bei dem gesättigten polyglycolisierten Glycerid um Lauroylpolyoxyl-32-glycerid handelt.

5. Feste Dispersion gemäß Anspruch 1, wobei die Zubereitung der festen Dispersion eine Kapselzubereitung ist.

6. Pharmazeutische Zubereitung, umfassend: die feste Dispersion gemäß Anspruch 1 und einen pharmazeutisch annehmbaren Arzneimittelhilfsstoff.

7. Pharmazeutische Zubereitung gemäß Anspruch 6, wobei die Zubereitung eine Kapsel- oder Tablettenzubereitung ist.

8. Feste Dispersion gemäß Anspruch 1, wobei die feste Dispersion Folgendes umfasst: 100 Gewichtsteile des 20-O-β-D-Glucopyranosyl-20(S)-protopanaxadiols und 200 bis 5000 Gewichtsteile des gesättigten polyglycolisierten Glycerids; und
wobei es sich bei dem gesättigten polyglycolisierten Glycerid um Lauroylpolyoxyl-32-glycerid handelt.

9. Pharmazeutische Zubereitung, umfassend: die feste Dispersion gemäß Anspruch 3 und einen pharmazeutisch annehmbaren Arzneimittelhilfsstoff.

## Revendications

1. Dispersion solide, comprenant :
du 20-O-β-D-glucopyranosyl-20(S)-protopanaxadiol comme principe pharmacologiquement actif ; et
un glycéride polyglycolisé saturé comme matrice lipidique.

2. Dispersion solide selon la revendication 1, dans laquelle le glycéride polyglycolisé saturé est un mélange de mono-, di- et triglycérides et de mono- et diesters de polyéthylène glycol.

3. Dispersion solide selon la revendication 1, dans laquelle la dispersion solide comprend 100 parties en poids du 20-O-β-D-glucopyranosyl-20(S)-protopanaxadiol et de 200 à 5000 parties en poids du glycéride polyglycolisé saturé.

4. Dispersion solide selon la revendication 1, dans laquelle le glycéride polyglycolisé saturé est le lauroyl macrogol-32 glycéride.

5. Dispersion solide selon la revendication 1, dans laquelle la formulation de la dispersion solide est une formulation de capsules.

6. Formulation pharmaceutique, comprenant la dispersion solide selon la revendication 1 et un excipient pharmaceutiquement acceptable.

7. Formulation pharmaceutique selon la revendication 6, dans laquelle la formulation est une formulation de capsules ou de comprimés.

8. Dispersion solide selon la revendication 1, dans laquelle la dispersion solide comprend 100 parties en poids du 20-O-β-D-glucopyranosyl-20(S)-protopanaxadiol et de 200 à 5000 parties en poids du glycéride polyglycolisé saturé, et
dans laquelle le glycéride polyglycolisé saturé est le lauroyl macrogol-32 glycéride.

9. Formulation pharmaceutique, comprenant la dispersion solide selon la revendication 3 et un excipient pharmaceutiquement acceptable.
